# EUROPÄISCHE PATENTSCHRIFT

(11) **EP 2 760 516 B1**
(45) Veröffentlichungstag und Bekanntmachung des Hinweises auf die Patenterteilung: **06.07.2016**
(21) Anmeldenummer: 12770082.1
(22) Anmeldetag: 27.09.2012
(51) Int. Cl.: A61M 16/16

(54) **ATEMLUFTBEFEUCHTER**
RESPIRATORY HUMIDIFIER
HUMIDIFICATEUR D'AIR INHALÉ

(30) Priorität: 01.10.2011 DE 102011054136
(43) Veröffentlichungstag der Anmeldung: 06.08.2014
(73) Patentinhaber: Hamilton Bonaduz AG, 7402 Bonaduz (CH)
(72) Erfinder: BÜCHI, Rudolf, 7000 Chur (CH)
(74) Vertreter: Caspary, Karsten
(86) Internationale Anmeldenummer: PCT/EP2012/069139
(87) Internationale Veröffentlichungsnummer: WO 2013/045586

(56) Entgegenhaltungen:
- US-A1- 2005 247 314
- US-A1- 2006 237 005
- US-A1- 2009 194 106
- US-A1- 2010 170 510
- US-B1- 6 554 260
- US-B1- 7 327 949

## Beschreibung

Die vorliegende Erfindung betrifft einen Atemluftbefeuchter für ein Beatmungssystem.

Bei der maschinellen Beatmung von Patienten, die beispielsweise auf einer Intensivstation liegen, wird der zu beatmende Patient mit Hilfe eines Beatmungsschlauchsystems pneumatisch mit dem Beatmungsgerät verbunden. Da das Atemgas, das dem Patienten zugeführt wird, hinsichtlich Temperatur und Feuchtigkeit den physiologischen Erfordernissen des Patienten angepasst werden muss, wird ein Atemluftbefeuchter in dem Einatem- oder Inspirationsschlauch angeordnet, der das Atemgas anwärmt und anfeuchtet. Der Atemluftbefeuchter weist einen mit destilliertem Wasser gefüllten Flüssigkeitsbehälter auf, durch den das Einatemgas geleitet und mit Feuchtigkeit angereichert wird.

Die Aufheizung der Flüssigkeit in dem Flüssigkeitsbehälter wird normalerweise über eine Heizplatte im unteren Abschnitt des Gehäuses des Atemluftbefeuchters bewirkt, wobei die Wärmeübertragung thermisch von der Heizplatte auf den wärmeleitfähigen Boden des Flüssigkeitsbehälters erfolgt. Die Temperatur des Atemgases wird beispielsweise beim Einströmen und beim Ausströmen durch geeignete Sensoren gemessen und zur Steuerung des Atemluftbefeuchters ausgewertet.

Damit der Flüssigkeitsbehälter nicht trocken läuft oder mit zu viel Flüssigkeit gefüllt wird, wird der Füllstand im Flüssigkeitsbehälter überwacht.

Da das Beatmungsschlauchsystem und die Flüssigkeitsbehälter aus Hygienegründen als medizinische Einmal- bzw. Wegwerfartikel ausgebildet sind, sind bei einem Atemluftbefeuchter zwei unterschiedliche Verwendungsabschnitte vorhanden. Zum einen das stationäre Gehäuse, das an die Stromversorgung und gegebenenfalls an andere medizinische Geräte über eine Datenleitung angeschlossen ist, und zum anderen der austauschbare Flüssigkeitsbehälter, der üblicher Weise bereits mit den Inspirationsschläuchen zusammengesteckt geliefert wird, um dem Personal möglichst wenige Möglichkeiten zu bieten, Anschlüsse fehlerhaft zu belegen.

In Betrieb müssen Gehäuse und Flüssigkeitsbehälter fest miteinander verbunden sein. Insbesondere ist für eine ausreichende Wärmeübertragung eine gute thermische Verbindung der Bodenplatte des Flüssigkeitsbehälters mit der Heizplatte des Gehäuses unbedingt erforderlich.

Bekannte Atemluftbefeuchter mit integriertem Flüssigkeitsbehälter wie beispielsweise in der US 2006/0113690 A1 beschrieben, weisen auch Benutzeroberflächen auf, die den Benutzer unter anderem über die Funktionsparameter des Atemluftbefeuchters informieren und zur Steuerung des Atemluftbefeuchters entsprechende Bedienelemente aufweisen. Diese Benutzeroberflächen sind jedoch meist senkrecht oder waagerecht angeordnet, so dass sie im Wesentlichen nur von vorne bzw. von oben eingesehen und bedient werden können. In einigen Fällen sind sie unterhalb des Flüssigkeitsbehälters angeordnet, wodurch sie zum Ablesen oder Einstellen schlecht zugänglich sind.

Weiterer Anordnungen der Flüssigkeitsbehälters in einem Atemluftbefeuchter sind in der US 4,715,998, US 2006/0237005, US 2009/0194106 und US 7327949 beschrieben.

Ein weiterer Nachteil von bekannten Atemluftbefeuchtern ist, dass sie relativ groß und unförmig, beispielsweise mit vielen Ecken und Kanten, aufgebaut sind, eine Vielzahl von Anschlüssen aufweisen und komplexe Bauelemente enthalten. Dadurch ist die Bedienung derartiger Geräte kompliziert und störanfällig sowie ihre Anschaffung teuer.

Es ist daher die Aufgabe der vorliegenden Erfindung, einen Atemluftbefeuchter bereitzustellen, der die oben genannten Nachteile überwindet und insbesondere eine sowohl von vorne als auch von oben bedienbare Benutzerschnittstelle aufweist, ergonomisch, einfach und kompakt aufgebaut ist und eine Mehrzahl von Funktionen zuverlässig erfüllt.

Diese Aufgabe wird durch die Merkmale des Anspruchs 1 gelöst. Vorteilhafte Ausgestaltungen und Ausführungsformen sind in den Unteransprüchen beschrieben.

Erfindungsgemäß wird ein Atemluftbefeuchter für ein Beatmungssystem bereitgestellt mit einem Gehäuse, das im Wesentlichen L-förmig mit einem senkrechten und einem waagerechten Schenkel und einem sich vom freien Ende des senkrechten Schenkels in Richtung des waagerechten Schenkels erstreckenden vorsprungartigen Abschnitt ausgebildet ist und eine Steuereinrichtung, eine Benutzeroberfläche und eine Heizplatte aufweist, wobei die Benutzeroberfläche auf dem vorsprungartigen Abschnitt und die Heizplatte auf dem waagerechten Schenkel angeordnet ist, und einem Flüssigkeitsbehälter, der im Wesentlichen U-artig einen Ausschnitt und eine Bodenplatte sowie zwei Anschlusselemente zur Verbindung mit je einem Beatmungsschlauch aufweist, wobei zur Beheizung der Flüssigkeit im Flüssigkeitsbehälter die Heizplatte erwärmbar und die Bodenplatte in Kontakt mit der Heizplatte bringbar ist, wobei der Flüssigkeitsbehälter derart auf das Gehäuse in einem Betriebszustand schiebbar ist, dass im Betriebszustand der vorsprungartige Abschnitt oberhalb der Bodenplatte angeordnet ist. Diese Konstruktion ist relativ einfach und kompakt und bietet dem Benutzer die ergonomisch zweckmäßige Möglichkeit; den Atemluftbefeuchter sowohl von oben als auch von vorne bedienen und seine Funktionsparameter einsehen zu können. Durch den Verzicht auf zusätzliche oder komplexe Elemente ist der Atemluftbefeuchter wenig störanfällig und auch Bedienfehler sind durch den einfachen Schiebmechanismus weniger wahrscheinlich.

Bevorzugt ist, dass im Betriebszustand das Gehäuse und der Flüssigkeitsbehälter gemeinsam eine im Wesentlichen durchgängige schräge Deckelfläche und eine im Wesentlichen durchgängige Mantelfläche des Atemluftbefeuchters bilden, wobei die Benutzeroberfläche in die schräge Deckelfläche integriert ist. Dies dient der Benutzerfreundlichkeit, da bis auf die Anschlusselemente keine größeren Öffnungen in der kompakten, leicht verstaubaren Einheit vorhanden sind.

Mit besonderem Vorteil weist das Gehäuse erste Befestigungsmittel und der Flüssigkeitsbehälter zweite Befestigungsmittel auf, wobei das Schieben des Flüssigkeitsbehälters auf das Gehäuse derart ein Ineinandergreifen der ersten und zweiten Befestigungsmittel bewirkt, dass die Bodenplatte von oben auf die Heizplatte gedrückt wird. Da ein vollständiger thermischer und ggf. auch elektrischer Kontakt zwischen der Heizplatte und der Bodenplatte für eine reibungslose Funktion des Atemluftbefeuchters essentiell ist, muss die Bodenplatte in irgendeiner Weise auf die Heizplatte gedrückt und in der Position gehalten werden. Dies wird durch das Ineinandergreifen der ersten und zweiten Befestigungsmittel gewährleistet. Eine derartige Befestigungskonstruktion ist wiederum einfach und kommt ohne aufwändige Mechanismen an der Heizplatte wie aus dem Stand der Technik bekannt aus.

In vorteilhafter Weise sind die ersten Befestigungsmittel im unteren Bereich des vorsprungartigen Abschnitts angeordnet. Dort sind sie wenig störend, vor Manipulationen geschützt und von einem Benutzer kaum einsehbar.

Des Weiteren ist bevorzugt, dass der Flüssigkeitsbehälter im Wesentlichen in einer Richtung parallel zu der Heizplatte auf das Gehäuse schiebbar ist.

Mit weiterem Vorteil erfolgt das Ineinandergreifen der ersten und zweiten Befestigungsmittel mittels eines lösbaren Rastmechanismus. Dieser Rastmechanismus ist einfach, kommt ohne zusätzliche Befestigungsmittel aus, die von einem Benutzer bedient werden müssen, und ermöglicht so das einfache Austauschen des üblicherweise als Einmal-/Wegwerfartikel ausgebildeten Flüssigkeitsbehälters.

Dabei ist es von Vorteil, wenn der Rastmechanismus ein Nut-Feder-Mechanismus ist, wobei die ersten Befestigungsmittel mindestens eine Feder und die zweiten Befestigungsmittel mindestens eine Nut aufweisen. Das Gehäuse weist dazu in vorteilhafter Weise mindestens eine Öffnung auf, durch die die mindestens eine Feder nach unten aus dem Gehäuse herausragt. Eine derartige Konstruktion ist mechanisch äußerst einfach, jedoch von außen kaum sichtbar und insbesondere im Betriebszustand des Atemluftbefeuchters nicht für den Benutzer sichtbar.

Vorteilhafterweise ist die mindestens eine Feder als einstückiges Federblechelement ausgebildet. Federblechmaterial ist kostengünstig sowie auf einfache Weise in die gewünschte Form zu bringen. Darüber hinaus weist es eine solide und kaum störanfällige Federfunktion auf, die zu einem langlebigen Betrieb des Atemluftbefeuchters beiträgt.

Bevorzugt weist der Flüssigkeitsbehälter im vorderen, vom senkrechten Schenkel des Gehäuses wegweisenden Bereich eine Griffeinrichtung auf. Damit kann der Flüssigkeitsbehälter auf intuitive Weise vom Benutzer aus dem stationären Gehäuse entfernt werden, und ein Benutzer wird den Griff ebenfalls beim Hineinschieben auf das Gehäuse verwenden. Die Griffeinrichtung ist bevorzugt als Ausnehmung in der Mantelfläche des Flüssigkeitsbehälters vorne ausgebildet und stellt so den einzigen Abschnitt auf der Mantelfläche des Atemluftbefeuchters dar, der nicht durchgängig ist. Dies führt dazu, dass der Benutzer intuitiv dahin geführt wird, diesen Griff zu verwenden, um den Flüssigkeitsbehälter vom feststehenden Gehäuse zu entfernen oder ihn wieder einzuschieben.

Weiter bevorzugt umfasst die Benutzeroberfläche des Atemluftbefeuchters Anzeigeelemente und Bedienelemente. Die Funktionen des Atemluftbefeuchters und gegebenenfalls von anderen Einheiten des Beatmungssystems können auf der Benutzeroberfläche angezeigt und auch bedient werden. Sämtliche Funktionsbestandteile des erfindungsgemäßen Atemluftbefeuchters, die der Benutzer im Betriebszustand in irgendeiner Weise handhaben kann, sind damit von der Deckelfläche des Atemluftbefeuchters zugänglich. Dies gilt insbesondere auch für die Anschlusselemente der Beatmungsschläuche und den Nachfüllschlauch.

Mit weiterem Vorteil ist auf der Rückseite des senkrechten Schenkels des Atemluftbefeuchters mindestens ein Anschluss zur Stromversorgung, Datenkommunikation oder dergleichen vorgesehen. Dabei ist der mindestens eine Anschluss mit Vorteil derart ausgebildet, dass er nicht über die im Wesentlichen durchgängige Mantelfläche des Atemluftbefeuchters hinausragt. Der Atemluftbefeuchter bietet dadurch an seinen Außenflächen keine hervorstehenden Angriffspunkte, die bei einer Positionierung des gesamten Gerätes oder bei einer Bewahrung stören könnten. Dies gilt auch für den Fall, dass kein Flüssigkeitsbehälter montiert ist.

Die Erfindung soll nachfolgend anhand von Ausführungsbeispielen unter Bezugnahme auf die beigefügten Figuren detailliert erläutert werden, in denen:
- Fig. 1: eine perspektivische Darstellung eines Gehäuses einer bevorzugten Ausführungsform des erfindungemäßen Atemluftbefeuchters zeigt;
- Fig. 2: eine perspektivische Darstellung eines Flüssigkeitsbehälters einer bevorzugten Ausführungsform des erfindungemäßen Atemluftbefeuchters zeigt;
- Fig. 3: eine perspektivische Darstellung des erfindungsgemäßen Atemluftbefeuchters der bevorzugten Ausführungsform im Betriebszustand zeigt;
- Fig. 4: eine perspektivische Ansicht eines Ausschnitts des Gehäuses des erfindungsgemäßen Atemluftbefeuchters gemäß der bevorzugten Ausführungsform zeigt; und die
- Fig. 5-9: den Rastmechanismus der bevorzugten Ausführungsform des erfindungsgemäßen Atemluftbefeuchters in unterschiedlichen Positionen des Flüssigkeitsbehälters zeigen.

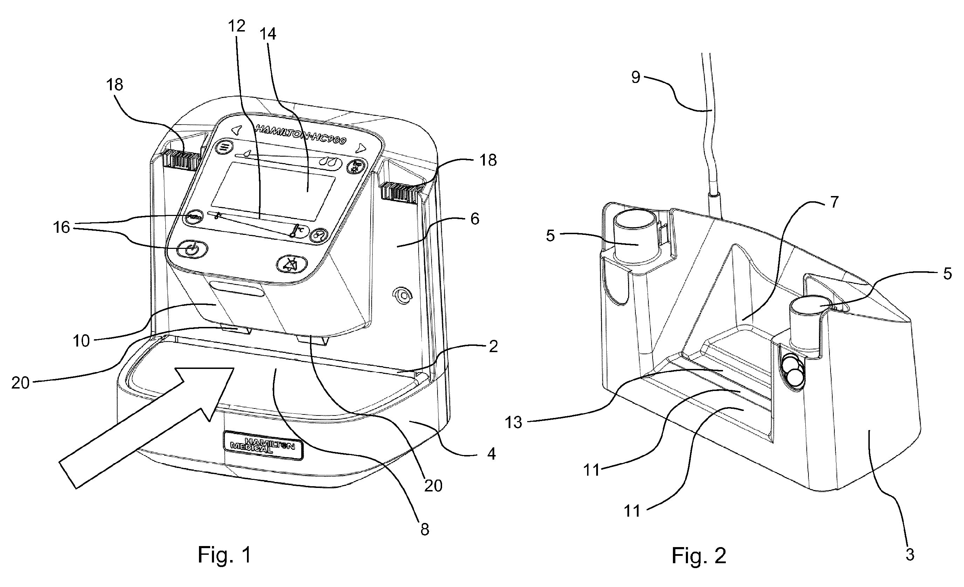

Fig. 1 zeigt in perspektivischer Ansicht das Gehäuse 2 eines erfindungsgemäßen Atemluftbefeuchters gemäß einer bevorzugten Ausführungsform. Das Gehäuse 2 weist im Wesentlichen eine L-Form mit einem waagrechten Schenkel 4 und einem senkrechten Schenkel 6 auf. An dem waagrechten Schenkel 4 ist eine Heizplatte 8 angeordnet, die im Wesentlichen waagrecht ausgerichtet ist und fast die gesamte nach oben gerichtete Fläche des waagrechten Schenkels 4 bedeckt. Vom oberen, freien Ende des senkrechten Schenkels 6 erstreckt sich in etwa mittig ein vorsprungartiger Abschnitt 10, dessen Oberfläche eine Benutzerschnittstelle oder Benutzeroberfläche 12 umfasst. Die Benutzerschnittstelle 12 weist eine Anzeigeeinrichtung 14 und Bedienelemente 16 auf, mittels derer sich der Atemluftbefeuchter überwachen und steuern lässt. Seitlich bezüglich des vorsprungartigen Abschnitts 10 versetzt sind am oberen Ende des senkrechten Schenkels 6 jeweils elektrische Kontaktelemente 18 angeordnet, die mit entsprechenden Verbindungsstücken eines Beatmungsschlauchsystems in elektrischen Kontakt gebracht werden können. Am unteren Ende des vorsprungartigen Abschnitts 10 sind zwei Federelemente 20 angeordnet, die jeweils aus einer (in Fig. 1 nicht sichtbaren) Öffnung nach unten heraus ragen.

Fig. 2 zeigt in perspektivischer Darstellung einen Flüssigkeitsbehälter 3 des erfindungsgemäßen Atemluftbefeuchters gemäß der bevorzugten Ausführungsform der Erfindung. Im oberen Bereich weist der Flüssigkeitsbehälter 3 je ein Anschlusselement 5 auf, das als Rohrstutzen mit kreisförmigem Querschnitt zur pneumatischen Verbindung mit einem Beatmungsschlauchsystem ausgebildet ist. Zwischen den beiden Anschlusselementen 5 weist der Flüssigkeitsbehälter 3 eine Ausnehmung bzw. einen Ausschnitt 7 auf. Des Weiteren ist auf der Deckelfläche des Flüssigkeitsbehälters 3 ein Nachfüllschlauch 10 angeordnet. Der Boden des Flüssigkeitsbehälters 3 ist als (in Fig. 2 nicht sichtbare) Bodenplatte aus Aluminium ausgebildet. Im unteren Bereich des Ausschnitts 7 sind parallel zur Richtung der Geraden zwischen den beiden Anschlusselementen 5 mehrere parallele Auflageflächen 11 ausgebildet, die etwa in der Mitte des unteren Bereichs des Ausschnitts 7 eine Nut 13 bilden, die senkrecht zu einer Befestigungsrichtung des Flüssigkeitsbehälters 3 ausgerichtet ist.

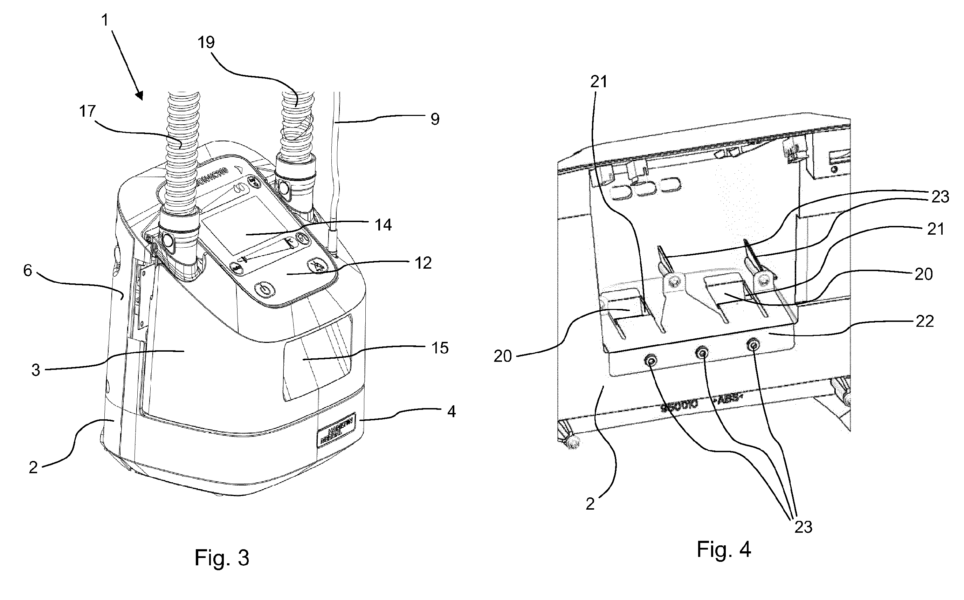

In Fig. 3 ist in perspektivischer Darstellung ein Atemluftbefeuchter 1 gemäß der bevorzugten Ausführungsform der Erfindung im Betriebszustand dargestellt. Dabei ist der Flüssigkeitsbehälter 3 in seiner Endstellung auf das Gehäuse 2 aufgeschoben. Man erkennt, dass die Deckelfläche des Flüssigkeitsbehälters 3 sowie die Benutzerschnittstelle 12 eine im Wesentlichen durchgängige Deckelfläche des Atemluftbefeuchters 1 bilden, die lediglich durch die aufgesteckten Beatmungsschläuche 17 und 19 unterbrochen ist. In Fig. 3 ebenfalls sichtbar ist der Griff 15 am vorderen Abschnitt des Flüssigkeitsbehälters 3, der gegriffen werden kann, um den Flüssigkeitsbehälter 3 aufzuschieben oder entsprechend aus dem Atemluftbefeuchter 1 zu entfernen.

Fig. 4 zeigt in perspektivischer Ansicht einen Ausschnitt des Gehäuses des erfindungsgemäßen Atemluftbefeuchters gemäß der bevorzugten Ausführungsform, wobei der Fokus dieser Abbildung auf dem ersten Befestigungsmittel liegt, das von innen an dem unteren Bereich des vorsprungartigen Abschnitts 10 im Inneren des Gehäuses 2 angeordnet ist. Das erste Befestigungsmittel ist als Federblech 22 ausgebildet, das mit mehreren Befestigungselementen 23 am Gehäuse 2 befestigt und zwei Federlaschen 20 aufweist, die im Wesentlichen V-förmig von innen jeweils durch eine Öffnung 21 im Gehäuse 2 nach unten ragen. Die Enden der Federlaschen 20 liegen frei, so dass es möglich ist, die V-förmigen Abschnitte von unten nach oben in den Innenraum des Gehäuses 2 zu drücken. Dabei verrasten die V-förmigen Federlaschen 20 in die Nut 13 ein, die man beispielsweise in Fig. 2 sieht. In der bevorzugten Ausführungsform weist das erste Befestigungsmittel zwei Federlaschen auf. Es ist anzumerken, dass dabei auch nur eine oder mehr als zwei Federelemente verwendet werden können, die auch nicht integral aus einem Stück Federblech gebildet sein müssen.

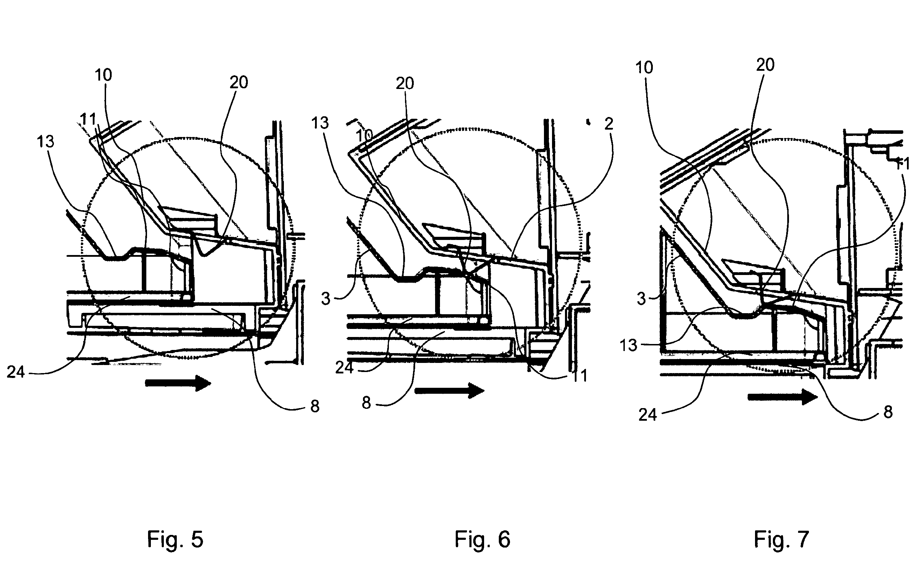

Anhand der Figuren 5 bis 9 soll nun der Rastmechanismus der bevorzugten Ausführungsform des erfindungsgemäßen Atemluftbefeuchters beschrieben werden. Fig. 5 zeigt in einem Detailausschnitt im Querschnitt eine Ausgangsposition beim Einschieben des Flüssigkeitsbehälters 3 in das Gehäuse 2. Im unteren Abschnitt erkennt man die Heizplatte 8, auf die die Bodenplatte 24 des Flüssigkeitsbehälters 3 aufgeschoben und schließlich mit ihr thermisch und elektrisch gekoppelt wird. Die Auflageflächen 11 des Flüssigkeitsbehälters 3 kommen zuerst mit der oder den Federlaschen 20 in Kontakt, die im entspannten Zustand aus der Öffnung 21 in den Zwischenraum zwischen dem vorsprungartigen Abschnitt 10 und der Heizplatte 8 ragen.

Fig. 6 zeigt den Flüssigkeitsbehälter 3 in einer weiter in das Gehäuse 2 eingeschobenen Stellung. Der Pfeil nach rechts zeigt in den Fig. 5 bis 8 die Einschieberichtung an. Man erkennt, dass die Federlasche 20 in Kontakt mit der Auflagefläche 11 ist, die bezüglich der Bodenplatte 24 schräg angeordnet ist und somit die Federlasche 20 bei Bewegung des Flüssigkeitsbehälters 3 nach rechts gegen ihre Federkraft nach oben in die Öffnung 21 hinein drückt.

Fig. 7 zeigt eine Position des Flüssigkeitsbehälters 3, der bezogen auf die Position in Fig. 6 noch weiter nach rechts verschoben ist. Die Federlasche 20 ist in maximaler Auslenkung nach oben in das Innere des vorsprungartigen Abschnitts 10 gebogen und liegt auf der im Wesentlichen waagerechten Auflagefläche 11 auf.

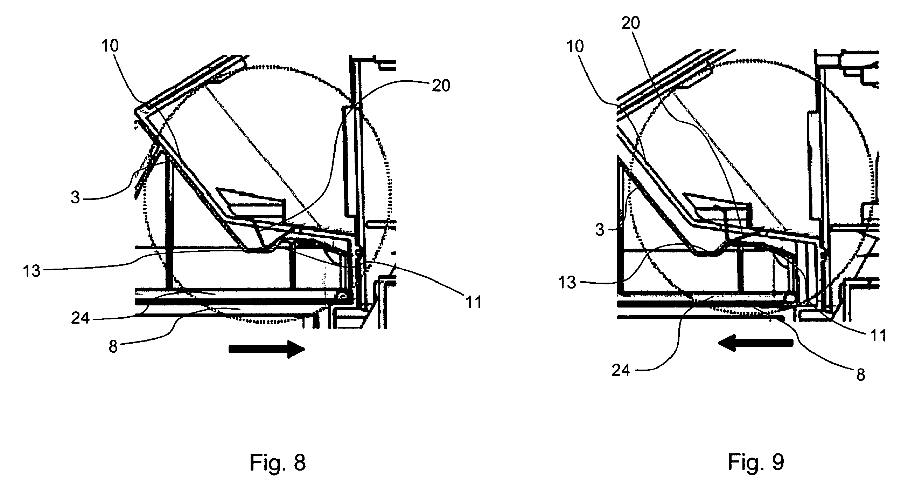

Beim weiteren Einschieben des Flüssigkeitsbehälters 3 nach rechts erreicht man die in Fig. 8 dargestellte Endposition. Die Federlasche 20 ist nunmehr bezogen auf die in Fig. 7 dargestellten Position etwas entspannt, weil sie entlang einer weiteren Auflagefläche 11, die schräg nach unten in Richtung der Nut 13 weist, gerutscht ist. Die Nut 13 bzw. die entsprechende Auflagefläche 11 ist nun im Formschluss mit der Federlasche 20, die noch einen Restdruck auf die Nut 13 und damit den Flüssigkeitsbehälter 3 ausübt, so dass die Bodenplatte 24 des Flüssigkeitsbehälters 3 auf die Heizplatte 8 gedrückt wird und nur durch Aufbringen einer vorbestimmten Lösekraft den Flüssigkeitsbehälter 3 wieder freigeben kann.

In Fig. 9 ist die Position des Flüssigkeitsbehälters 3 dargestellt, in der die Federkraft der Federlasche 20 im Wesentlichen bereits überwunden ist, da der Flüssigkeitsbehälter 3 bereits ein gutes Stück mit Hilfe des Griffs 15 aus dem Gehäuse 2 herausgezogen wurde (Pfeilrichtung nach links). Auch hier ist wieder sichtbar, dass sich die Federlasche 20 nach oben in das Innere des vorsprungartigen Abschnitts 10 bewegt gegen ihre Federkraft. Der Fachmann wird verstehen, dass ein weiteres Herausziehen des Flüssigkeitsbehälters 3 aus dem Gehäuse 2 die vollständige Freigabe des Federelements bewirkt, das dann nicht mehr im Eingriff mit der Oberfläche des Flüssigkeitsbehälters 3 steht.

Im Rahmen dieser Erfindung sind anstatt einer Nut- und Federkonstruktion mit einem Federblech noch andere Mechanismen und Konstruktionen für die ersten und zweiten Befestigungsmittel denkbar, beispielsweise mittels Magneten.

Mit dem Gegenstand der vorliegenden Erfindung wurde ein Atemluftbefeuchter bereitgestellt, der einfach und kompakt aufgebaut ist, eine von oben und gegebenenfalls auch von vorne bedienbare Benutzerschnittstelle aufweist und der eine Mehrzahl von Funktionen zuverlässig erfüllt.

## Patentansprüche

1. Atemluftbefeuchter (1) für ein Beatmungssystem mit
einem Gehäuse (2), das mit einem waagerechten und einem senkrechten Schenkel (4, 6) und einem sich vom freien Ende des senkrechten Schenkels (6) in Richtung des waagerechten Schenkels (4) erstreckenden vorsprungartigen Abschnitt (10) ausgebildet ist und eine Steuereinrichtung, eine Benutzeroberfläche (12) und eine Heizplatte (8) aufweist, wobei die Benutzeroberfläche (12) auf dem vorsprungartigen Abschnitt (10) und die Heizplatte (8) auf dem waagerechten Schenkel (4) angeordnet ist, und
einem Flüssigkeitsbehälter (3), der einen Ausschnitt (7) und eine Bodenplatte (24) sowie zwei Anschlusselemente (5) zur Verbindung mit je einem Beatmungsschlauch aufweist,
wobei zur Beheizung der Flüssigkeit im Flüssigkeitsbehälter (3) die Heizplatte (8) erwärmbar und die Bodenplatte (24) in Kontakt mit der Heizplatte (8) bringbar ist,
wobei der Flüssigkeitsbehälter (3) derart auf das Gehäuse (2) in einem Betriebszustand schiebbar ist, dass im Betriebszustand der vorsprungartige Abschnitt (10) oberhalb der Bodenplatte (24) angeordnet ist,
**dadurch gekennzeichnet, dass**
das Gehäuse (2) erste Befestigungsmittel (20, 22) und der Flüssigkeitsbehälter (3) zweite Befestigungsmittel (13) aufweist, wobei das Schieben des Flüssigkeitsbehälters (3) auf das Gehäuse (2) derart ein Ineinandergreifen der ersten (20, 22) und zweiten Befestigungsmittel (13) bewirkt, dass die Bodenplatte (24) von oben auf die Heizplatte (8) gedrückt wird,
wobei die ersten Befestigungsmittel (20, 22) im unteren Bereich des vorsprungartigen Abschnitts (10) angeordnet sind.

2. Atemluftbefeuchter (1) nach Anspruch 1, **dadurch gekennzeichnet, dass** im Betriebszustand das Gehäuse (2) und der Flüssigkeitsbehälter (3) gemeinsam eine im Wesentlichen durchgängige schräge Deckelfläche und eine im Wesentlichen durchgängige Mantelfläche des Atemluftbefeuchters (1) bilden, wobei die Benutzeroberfläche (12) in die schräge Deckelfläche integriert ist.

3. Atemluftbefeuchter (1) nach Anspruch 1 oder 2, **dadurch gekennzeichnet, dass** der Flüssigkeitsbehälter (3) im Wesentlichen in einer Richtung parallel zu der Heizplatte (8) auf das Gehäuse (2) schiebbar ist.

4. Atemluftbefeuchter (1) nach einem der vorhergehenden Ansprüche, **dadurch gekennzeichnet, dass** das Ineinandergreifen mittels eines lösbaren Rastmechanismus erfolgt.

5. Atemluftbefeuchter (1) nach Anspruch 4, **dadurch gekennzeichnet, dass** der Rastmechanismus ein Nut-Feder-Mechanismus ist, wobei die ersten Befestigungsmittel mindestens eine Feder (20, 22) und die zweiten Befestigungsmittel mindestens eine Nut (13) aufweisen.

6. Atemluftbefeuchter (1) nach Anspruch 5, **dadurch gekennzeichnet, dass** das Gehäuse (2) mindestens eine Öffnung (21) aufweist, durch die die mindestens eine Feder (20) nach unten aus dem Gehäuse (2) heraus ragt.

7. Atemluftbefeuchter (1) nach Anspruch 5 oder 6, **dadurch gekennzeichnet, dass** die mindestens eine Feder (20) als einstückiges Federblechelement (22) ausgebildet ist.

8. Atemluftbefeuchter (1) nach einem der vorhergehenden Ansprüche, **dadurch gekennzeichnet, dass** der Flüssigkeitsbehälter (3) im vorderen, vom senkrechten Schenkel (6) des Gehäuses (2) weg weisenden Bereich eine Griffeinrichtung (15) aufweist.

9. Atemluftbefeuchter (1) nach einem der vorhergehenden Ansprüche, **dadurch gekennzeichnet, dass** die Benutzeroberfläche (12) Anzeigeelemente (14) und Bedienelemente (16) umfasst.

10. Atemluftbefeuchter (1) nach einem der vorhergehenden Ansprüche, **dadurch gekennzeichnet, dass** auf der Rückseite des senkrechten Schenkels (6) mindestens ein Anschluss zur Stromversorgung, Datenkommunikation oder dergleichen vorgesehen ist.

11. Atemluftbefeuchter (1) nach Anspruch 10, **dadurch gekennzeichnet, dass** der mindestens eine Anschluss derart ausgebildet ist, dass er nicht über die im Wesentlichen durchgängige Mantelfläche des Atemluftbefeuchters (1) hinaus ragt.

## Claims

1. A respiratory humidifier (1) for a ventilation system having
a housing (2), which is designed with a horizontal part and a vertical part (4, 6) and a projecting portion (10) extending from the free end of the vertical part (6) toward the horizontal part (4), and which comprises a control unit, a user interface (12), and a heating plate (8), wherein the user interface (12) is arranged on the projecting portion (10) and the heating plate (8) is arranged on the horizontal part (4); and
a liquid container (3) comprising a recess (7) and a bottom plate (24) as well as two connector elements (5), each of which adapted to establish a connection with a breathing tube;
wherein the heating plate (8) is adapted to be heated for heating the liquid in the liquid container (3) and wherein the bottom plate (24) is adapted to be brought into contact with the heating plate (8);
wherein the liquid container (3) is slidable onto the housing (2) to establish an operating state in such a way that, when in said operating state, the projecting portion (10) of the housing (2) is positioned above the bottom plate (24) of the liquid container (3)
**characterized in that**
the housing (2) comprises first fastening means (20, 22), and the liquid container (3) comprises second fastening means (13), wherein the sliding of the liquid container (3) onto the housing (2) results in the engagement between the first (20, 22) and second fastening means (13) in such a way that the bottom plate (24) is pressed from above onto the heating plate (8);
wherein the first fastening means (20, 22) is arranged in the lower area of the projecting portion (10).

2. Respiratory humidifier (1) according to claim 1, **characterized in that** when the humidifier is in said operating state, the housing (2) and the liquid container (3) jointly form an essentially continuous, slanted top surface and an essentially continuous lateral surface of the respiratory humidifier (1), wherein the user interface (12) is integrated into the slanted top surface.

3. Respiratory humidifier (1) according to claim 1 or 2, wherein the liquid container (3) is slidable onto the housing (2) in a direction essentially parallel to the heating plate (8).

4. Respiratory humidifier (1) according to one of the previous claims, **characterized in that** the engagement occurs by means of a releasable latching mechanism.

5. Respiratory humidifier (1) according to claim 4, **characterized in that** the latching mechanism is a tongue-and-groove mechanism, wherein the first fastening means comprises at least one spring (20, 22), and wherein the second fastening means comprises at least one groove (13).

6. Respiratory humidifier (1) according to claim 5, wherein the housing (2) comprises at least one opening (21), through which the at least one spring (20) projects downward out of the housing (2).

7. Respiratory humidifier (1) according to claim 5 or 6, **characterized in that** the at least one spring (20) is formed as a one-piece spring plate element (22).

8. Respiratory humidifier (1) according to one of the previous claims, **characterized in that** the liquid container (3) comprises a gripping device (15) in the forward area facing away from the vertical part (6) of the housing (2).

9. Respiratory humidifier (1) according to one of the previous claims, **characterized in that** the user interface (12) comprises display elements (14) and operating elements (16).

10. Respiratory humidifier (1) according to one of the previous claims, **characterized in that** at least one port for a power supply, for data communications, etc., is provided on the rear of the vertical part (6).

11. Respiratory humidifier (1) according to claim 10, **characterized in that** the at least one port is designed in such a way that it does not project beyond the essentially continuous lateral surface of the respiratory humidifier (1).

## Revendications

1. Humidificateur d'air à respirer (1) pour un système respiratoire, comprenant
un boîtier (2), réalisé avec une branche horizontale et une branche verticale (4, 6) et avec une partie (10) en forme de saillie, qui s'étend de l'extrémité libre de la branche verticale (6) en direction de la branche horizontale (4), et un dispositif de commande, une interface utilisateur (12) et une plaque chauffante (8), l'interface utilisateur (12) étant disposée sur la partie (10) en forme de saillie et la plaque chauffante (8) étant disposée sur la branche horizontale (4), et
un réservoir de liquide (3), qui présente une partie (7) et une plaque de fond (24), ainsi que deux éléments de raccordement (5) pour le raccordement à un tuyau flexible respiratoire respectif,
la plaque chauffante (8) pouvant être chauffée pour le chauffage du liquide contenu dans le réservoir (3) et la plaque de fond (24) pouvant être amenée au contact de la plaque chauffante (8),
le réservoir de liquide (3) pouvant être poussé sur le boîtier (2) dans un état de fonctionnement de telle sorte que, dans cet état, la partie (10) en forme de saillie est disposée au-dessus de la plaque de fond (24), **caractérisé en ce que**
le boîtier (2) présente des premiers moyens de fixation (20, 22) et le réservoir de liquide (3) présente des deuxièmes moyens de fixation (13), la poussée du réservoir de liquide (3) sur le boîtier (2) provoquant une mise en prise des premiers (20, 22) et deuxièmes moyens de fixation (13), de telle sorte que la plaque de fond (24) est pressée à partir du haut sur la plaque chauffante (8),
les premiers moyens de fixation (20, 22) étant disposés dans la zone inférieure de la partie (10) en forme de saillie.

2. Humidificateur d'air à respirer (1) selon la revendication 1, **caractérisé en ce que**, dans l'état de fonctionnement, le boîtier (2) et le réservoir de liquide (3) forment conjointement une surface de couverture oblique essentiellement continue et une surface d'enveloppe essentiellement continue de l'humidificateur d'air à respirer (1), l'interface utilisateur (12) étant intégrée dans la surface de couverture oblique.

3. Humidificateur d'air à respirer (1) selon l'une des revendications 1 et 2, **caractérisé en ce que** le réservoir de liquide (3) peut être poussé sur le boîtier (2) essentiellement dans une direction parallèle à la plaque chauffante (8).

4. Humidificateur d'air à respirer (1) selon l'une des revendications précédentes, **caractérisé en ce que** la mise en prise s'effectue au moyen d'un mécanisme d'encliquetage réversible.

5. Humidificateur d'air à respirer (1) selon la revendication 4, **caractérisé en ce que** le mécanisme d'encliquetage est un mécanisme à rainure et languette, les premiers moyens de fixation présentant au moins une languette (20, 22) et les deuxièmes moyens de fixation présentant au moins une rainure (13).

6. Humidificateur d'air à respirer (1) selon la revendication 5, **caractérisé en ce que** le boîtier (2) présente au moins une ouverture (21), au travers de laquelle la au moins une languette (20) dépasse vers le bas du boîtier (2).

7. Humidificateur d'air à respirer (1) selon l'une des revendications 5 et 6, **caractérisé en ce que** la au moins une languette (20) est réalisée sous forme d'élément de tôle élastique (22) monobloc.

8. Humidificateur d'air à respirer (1) selon l'une des revendications précédentes, **caractérisé en ce que** le réservoir de liquide (3) présente un dispositif de préhension (15) dans la zone avant, orientée à l'écart de la branche verticale (6) du boîtier (2).

9. Humidificateur d'air à respirer (1) selon l'une des revendications précédentes, **caractérisé en ce que** l'interface utilisateur (12) comprend des éléments d'affichage (14) et des éléments de commande (16).

10. Humidificateur d'air à respirer (1) selon l'une des revendications précédentes, **caractérisé en ce qu'**au moins un raccord pour l'alimentation en courant, la communication de données, ou autres, est prévu sur le côté arrière de la branche verticale (6).

11. Humidificateur d'air à respirer (1) selon la revendication 10, **caractérisé en ce que** le au moins un raccord est réalisé de telle sorte qu'il ne dépasse pas de la surface d'enveloppe essentiellement continue de l'humidificateur (1).
